# FASCICULE DE BREVET EUROPEEN

(11) **EP 1 542 754 B1**
(45) Date de publication et mention de la délivrance du brevet: **09.12.2009**
(21) Numéro de dépôt: 03797354.2
(22) Date de dépôt: 19.09.2003
(51) Int. Cl.: A61M 15/00

(54) **Dispositif de distribution de produit fluide**
Vorrichtung zum Versprühen von Flüssigkeiten
Fluid product spraying device

(30) Priorité: 20.09.2002 FR 0211791
(43) Date de publication de la demande: 22.06.2005
(73) Titulaire: VALOIS S.A.S., 27110 Le Neubourg (FR)
(72) Inventeur: BRUNA, Pascal, F-76300 Sotteville les Rouen (FR); SAVALLE, Matthieu, F-76000 Rouen (FR)
(74) Mandataire: CAPRI
(86) Numéro de dépôt international: PCT/FR2003/002765
(87) Numéro de publication internationale: WO 2004/026379

(56) Documents cités:
- FR-A- 1 242 253
- US-A- 1 495 924
- US-A- 2 316 095
- US-A- 5 480 390
- US-A1- 2002 023 641

## Description

La présente invention concerne un dispositif de pulvérisation de produit fluide, et plus particulièrement un dispositif de pulvérisation nasale du type bidose, c'est-à-dire comportant deux doses de produit à distribuer.

Des dispositifs de pulvérisation du type bidose ont été développés pour de nombreuses applications, notamment dans le domaine de la pharmacie. Ces distributeurs sont utilisés notamment dans le domaine de la pulvérisation nasale. Ces dispositifs comportent généralement des réservoirs contenant deux doses de produit, chaque dose étant destinée à une narine respective.

Selon la nature du produit, particulièrement lorsqu'il s'agit d'un médicament, les conditions de remplissage et de stockage du produit peuvent être assez contraignantes. Ainsi, de nombreux produits fluides, c'est-à-dire liquide ou pulvérulent, du domaine pharmaceutique nécessitent un remplissage en zone stérile et un stockage en chambre froide. Dans les dispositifs de pulvérisation existants, le produit est généralement rempli après assemblage complet du dispositif, ce qui implique des machines de remplissage spécialement adaptées aux dispositifs en question, ces machines devant bien entendu être dans des zones stériles. Après remplissage, le dispositif dans son ensemble doit être stocké en chambre froide. Etant donné les coûts très élevés des surfaces en zones stériles et des volumes en chambres froides, l'utilisation de machines de remplissage spécifiques s'avère être un inconvénient du point de vue du coût, et il en est de même en ce qui concerne le stockage en chambre froide, le volume d'un dispositif de pulvérisation nasale étant souvent assez important.

Un dispositif de produit fluide est décrit dans le document US-A-1495924. La présente invention a pour but de fournir un dispositif de pulvérisation de produit fluide qui ne reproduit pas les inconvénients susmentionnés.

Ainsi, la présente invention a pour but de fournir un dispositif de pulvérisation de produit fluide pour lequel les surfaces et volumes de zones stériles lors du remplissage et de chambres froides lors du stockage sont minimales.

La présente invention a également pour but de fournir un tel dispositif de pulvérisation de produit fluide qui soit simple et peu coûteux à fabriquer et à assembler, et fiable dans son utilisation.

La présente invention est décrite dans la revendication et a donc pour objet un dispositif de pulvérisation de produit fluide.

Avantageusement, lesdits moyens d'accès latéral comprennent une fenêtre prévue dans une paroi latérale dudit corps.

Avantageusement, lesdits moyens d'accès latéral comprennent deux fenêtres diamétralement opposées.

Avantageusement, lesdits moyens de réception de réservoir comportent des moyens d'encliquetage du réservoir dans ledit corps.

Avantageusement, les moyens de pulvérisation comportent une tige déplaçable axialement coopérant avec le premier bouchon dudit réservoir.

Avantageusement, lesdits moyens d'ouverture de réservoir comportent des moyens de percement du second bouchon du réservoir.

Avantageusement, le corps, les moyens de pulvérisation, les moyens d'actionnement et les moyens d'ouverture de réservoir sont assemblés pour former une unité, le réservoir, après remplissage et bouchage, étant assemblé dans cette unité.

Avantageusement, lesdits moyens d'accès latéral comportent un couvercle amovible.

Avantageusement, le réservoir contient deux doses de produit, lesdits moyens de pulvérisation et/ou lesdits moyens d'actionnement comportant des moyens de fractionnement de dose, de sorte qu'à chaque actionnement du dispositif, une dose de produit est pulvérisée.

D'autres caractéristiques et avantages de la présente invention apparaîtront plus clairement au cours de la description détaillée suivante, faite en référence aux dessins joints, donnés à titre d'exemples non limitatifs, et sur lesquels :
- la figure 1 est une vue schématique en section transversale d'un réservoir de produit fluide destiné à un dispositif de pulvérisation de produit fluide selon un mode de réalisation avantageux de la présente invention ;
- la figure 2 est une vue schématique en section transversale d'un dispositif de pulvérisation de produit fluide selon un mode de réalisation avantageux de la présente invention, dans lequel s'adapte le réservoir de la figure 1 ;
- la figure 3 est une vue similaire à celle de la figure 2, le réservoir étant fixé à l'intérieur du dispositif ;
- la figure 4 est une vue similaire à celle de la figure 3, après actionnement du dispositif ; et
- la figure 5 est une vue schématique en perspective d'un dispositif de pulvérisation de produit fluide selon un mode de réalisation avantageux de la présente invention, montrant le dispositif et le réservoir séparément.

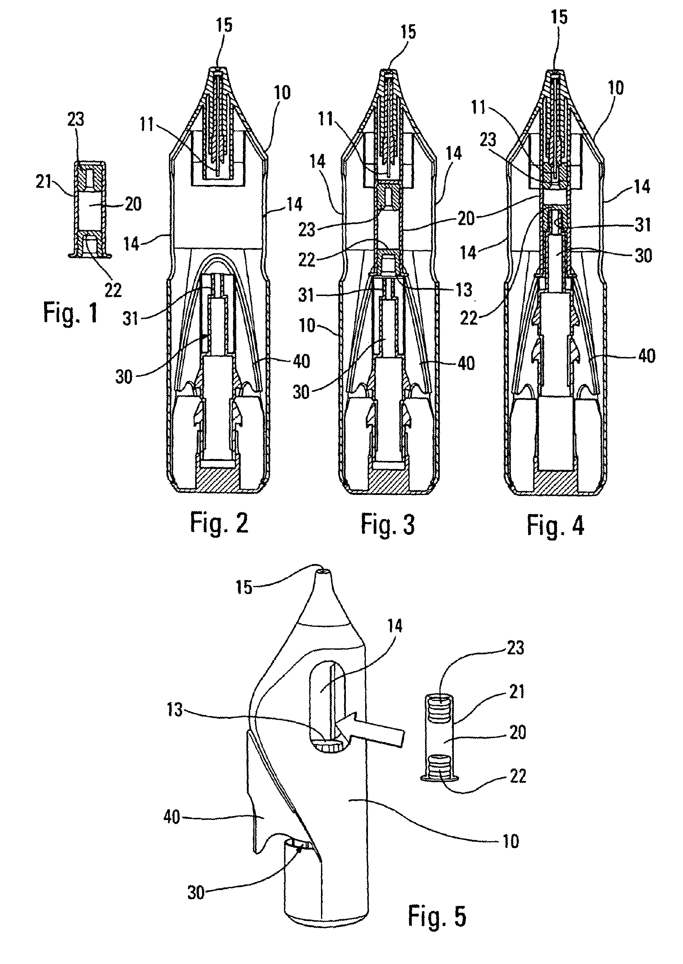

En référence à la figure 1, il est représenté un réservoir 20 qui s'adapte au dispositif de pulvérisation de produit fluide de la présente invention. Ce réservoir forme une unité étanche séparée du reste du dispositif, et est avantageusement constituée d'un tube creux 21 qui est bouché des deux côtés par un premier et un second bouchon d'étanchéité 22, 23. Ce type de réservoir peut être en verre, ou tout autre matériau approprié, et les bouchons 22, 23 peuvent être réalisés en matériau élastomère, ou tout autre matériau approprié pour obturer de manière étanche le contenu du réservoir 20 après son remplissage. Ce type de réservoir, notamment lorsqu'il ne contient que deux doses de produit, est donc de très petite dimension, ce qui limite les surfaces de zone stérile pour le remplissage et les volumes de chambre froide pour le stockage de ces réservoirs après le remplissage.

La figure 2 est une vue schématique d'un dispositif de pulvérisation dans lequel le réservoir de la figure 1 peut s'adapter. Ce dispositif comporte un corps 10 qui est pourvu d'un orifice de pulvérisation 15. Le corps 10 comporte des moyens d'ouverture 11 de réservoir 20, ces moyens pouvant par exemple être formés par un moyen de percement, tel qu'une aiguille. De préférence, cette aiguille 11 est fixe par rapport au corps 10 et disposée en regard de ou reliée à l'orifice de pulvérisation 15, cette aiguille étant destinée à percer le second bouchon 23 du réservoir 20 lors de l'actionnement du dispositif. Le dispositif de pulvérisation comporte en outre des moyens de pulvérisation 30, et des moyens d'actionnement 40 destinés à actionner lesdits moyens de pulvérisation. La pulvérisation est un aspect important, notamment pour les dispositifs de pulvérisation nasale, pour lesquels le produit doit être finement pulvérisée pour fournir une efficacité thérapeutique optimale. Avantageusement, les moyens de pulvérisation 30 comportent une tige, ou un élément similaire à une tige, déplaçable axialement, et qui est adaptée à coopérer avec le premier piston 22 du réservoir 20 pour déplacer celui-ci axialement à l'intérieur du réservoir 20. De manière connue, ce déplacement du premier bouchon 22 à l'intérieur du réservoir provoque une surpression dans celui-ci et donc un déplacement du second bouchon 23 en direction de l'aiguille 11 jusqu'à ce que celle-ci transperce le bouchon 23 et relie le contenu du réservoir 20 à l'orifice de pulvérisation 15. De préférence, les moyens d'actionnement 40 comportent un élément d'actionnement latéral, c'est-à-dire un élément qui se déplace dans une direction différente de la direction de déplacement des moyens de pulvérisation 30. Cet élément d'actionnement latéral 40 peut par exemple être réalisé par une patte pivotante montée sur le corps 10, et qui coopère avec les moyens de pulvérisation 30 pour déplacer ceux-ci lors de l'actionnement. Bien entendu, des moyens de pulvérisation et d'actionnement différents sont envisageables. Avantageusement, dans le cas d'un bidose, des moyens de fractionnement de doses sont prévus pour fractionner le contenu du réservoir en deux doses. Ces moyens de fractionnement de doses peuvent être réalisés sur les moyens de pulvérisation 30 et/ou sur les moyens d'actionnement 40. Ces moyens de fractionnement de doses ne seront pas décrits plus amplement en détail ci-après, car ils ne sont pas directement liés à la présente invention, et qu'ils peuvent être de forme quelconque.

Selon l'invention, le corps 10 comporte des moyens de réception 13 de réservoir et des moyens d'accès latéral 14. Ces moyens d'accès latéral 14 permettent un chargement du réservoir 20 à l'intérieur du corps 10 de manière latérale, de sorte que l'assemblage du réservoir à l'intérieur du corps peut être réalisé juste avant l'utilisation du dispositif. L'assemblage du réservoir est alors réalisé dans une unité complète formée par le corps, les moyens de pulvérisation, les moyens d'actionnement et les moyens d'ouverture du réservoir. Avantageusement, les moyens d'accès latéral comportent au moins une fenêtre 14 réalisée dans une paroi latérale du corps 10. Les figures montrent un corps 10 comportant deux fenêtres latérales 14 diamétralement opposées. Les moyens de réception de réservoir 13 peuvent être réalisés sous la forme de moyens d'encliquetage, ou tous autres moyens appropriés qui permettent de recevoir fixement le réservoir 20 à l'intérieur du corps 10. Bien entendu, des moyens de fixation ou de butée complémentaires (non représentés) peuvent être prévus pour favoriser une meilleure fixation de ce réservoir dans le corps. Eventuellement, un couvercle amovible (non représenté) pourrait être prévu au niveau de la ou des fenêtre(s) latérale(s) 14, ce couvercle pouvant être réalisée d'une façon quelconque souhaitable.

Le chargement latéral du réservoir dans le corps permet donc de réaliser un réservoir séparé, de le remplir et de le boucher de manière hermétique puis de le stocker indépendamment du reste du dispositif de pulvérisation. Le reste du dispositif peut de son côté être assemblé dans une zone non stérile et stocké dans un local qui n'est pas une chambre froide. Ce n'est que très peu avant l'utilisation du dispositif que le réservoir peut être assemblé dans le corps 10, le contenu du réservoir restant protégé par les bouchons étanches 22, 23 jusqu'au premier actionnement du dispositif. Le chargement latéral du réservoir présente l'avantage de pouvoir utiliser un actionnement latéral avec des moyens de pulvérisation se déplaçant de manière axiale, et de pouvoir assembler la totalité du dispositif, indépendamment du réservoir. L'assemblage du dispositif en est simplifié, et le dispositif lui-même est également simplifié, en économisant notamment une pièce, généralement nécessaire pour pré-assembler le réservoir lorsque celui-ci doit être assemblé de manière axiale à l'intérieur du corps. La présente invention permet donc de réaliser un dispositif de pulvérisation de produit fluide moins cher, de fonctionnement fiable, et pour lequel des économies importantes peuvent être réalisées lors du remplissage et du stockage.

Bien que l'invention ait été décrite en référence à un mode de réalisation particulier de celle-ci, il est entendu qu'un homme du métier peut y apporter toutes modifications, sans sortir du cadre de la présente invention tel que défini par les revendications annexées.

## Revendications

1. Dispositif de pulvérisation de produit fluide, comportant un corps (10) pourvu d'un orifice de pulvérisation (15), un réservoir (20) contenant le produit fluide à pulvériser, des moyens de pulvérisation (30) pour pulvériser en une ou plusieurs doses le produit contenu dans le réservoir (20), et des moyens d'actionnement (40) pour actionner lesdits moyens de pulvérisation (30), ledit réservoir (20) étant fermé de manière étanche avant le premier actionnement du dispositif de pulvérisation, le corps (10) comportant des moyens d'ouverture (11) du réservoir adaptés à ouvrir ledit réservoir (20) lors de l'actionnement du dispositif, ledit réservoir (20) formant une unité étanche séparée dudit corps (10), ledit réservoir (20) étant rempli avec le produit fluide et fermé hermétiquement avant son assemblage dans ledit corps (10), ledit corps (10) comportant des moyens de réception (13) de réservoir et des moyens d'accès latéral (14) pour assembler latéralement et fixer ledit réservoir (20) rempli dans ledit corps (10), **caractérisé en ce que** lesdits moyens d'actionnement (40) comportent un élément d'actionnement latéral déplaçable dans une direction différente de la direction de déplacement desdits moyens de pulvérisation (30).

2. Dispositif selon la revendication 1, dans lequel lesdits moyens d'accès latéral comprennent une fenêtre (14) prévue dans une paroi latérale dudit corps (10).

3. Dispositif selon la revendication 2, dans lequel lesdits moyens d'accès latéral comprennent deux fenêtres (14) diamétralement opposées.

4. Dispositif selon l'une quelconque des revendications précédentes, dans lequel lesdits moyens de réception (13) de réservoir comportent des moyens d'encliquetage du réservoir (20) dans ledit corps (10).

5. Dispositif selon l'une quelconque des revendications précédentes, dans lequel ledit réservoir (20) est formé par un tube creux (21) obturé de manière étanche par des premier et second bouchons (22, 23) disposés dans ledit tube (21), le produit fluide étant disposé entre lesdits premier et second bouchons (22, 23).

6. Dispositif selon la revendication 5, dans lequel les moyens de pulvérisation (30) comportent une tige (31) déplaçable axialement coopérant avec le premier bouchon (22) dudit réservoir (20).

7. Dispositif selon la revendication 5 ou 6, dans lequel lesdits moyens d'ouverture (11) de réservoir comportent des moyens de percement du second bouchon (23) du réservoir (20).

8. Dispositif selon l'une quelconque des revendications précédentes, dans lequel le corps (10), les moyens de pulvérisation (30), les moyens d'actionnement (40) et les moyens d'ouverture (11) de réservoir sont assemblés pour former une unité, le réservoir (20), après remplissage et bouchage, étant assemblé dans cette unité.

9. Dispositif selon l'une quelconque des revendications précédentes, dans lequel lesdits moyens d'accès latéral (14) comportent un couvercle amovible.

10. Dispositif selon l'une quelconque des revendications précédentes, dans lequel le réservoir (20) contient deux doses de produit, lesdits moyens de pulvérisation (30) et/ou lesdits moyens d'actionnement (40) comportant des moyens de fractionnement de dose, de sorte qu'à chaque actionnement du dispositif, une dose de produit est pulvérisée.

## Claims

1. A fluid spray device comprising: a body (10) provided with a spray orifice (15), a reservoir (20) containing the fluid to be sprayed, spray means (30) for spraying one or more doses of the fluid contained in the reservoir (20); and actuator means (40) for actuating said spray means (30), said reservoir (20) being closed in sealed manner before the spray device is actuated for the first time, the body (10) including reservoir opening means (11) adapted to open said reservoir (20) while the device is being actuated, said reservoir (20) forming a sealed unit that is separate from said body (10), said reservoir (20) being filled with fluid and being sealed hermetically before it is assembled in said body (10), and said body (10) including receiver means (13) for receiving the reservoir, and lateral access means (14) for enabling said filled reservoir (20) to be assembled sideways into said body (10) and to be secured therein, said device being **characterized in that** said actuator means (40) include a lateral actuator element that is displaceable in a direction that is different from the displacement direction of said spray means (30).

2. A device according to claim 1, in which said lateral access means comprise a window (14) provided in a side wall of said body (10).

3. A device according to claim 2, in which said lateral access means comprise two diametrally-opposite windows (14).

4. A device according to any preceding claim, in which said receiver means (13) for receiving the reservoir comprise snap-fastener means for snap-fastening the reservoir (20) in said body (10).

5. A device according to any preceding claim, in which said reservoir (20) is formed by a hollow tube (21) that is closed in sealed manner by first and second plugs (22, 23) disposed in said tube (21), the fluid being disposed between said first and second plugs (22, 23).

6. A device according to claim 5, in which the spray means (30) include an axially-displaceable rod (31) that co-operates with the first plug (22) of said reservoir (20).

7. A device according to claim 5 or claim 6, in which said reservoir opening means (11) include piercing means for piercing the second plug (23) of the reservoir (20).

8. A device according to any preceding claim, in which the body (10), the spray means (30), the actuator means (40), and the reservoir opening means (11) are assembled to form a unit, the reservoir (20) being assembled in said unit after said reservoir has been filled and plugged.

9. A device according to any preceding claim, in which said lateral access means (14) include a removable cover.

10. A device according to any preceding claim, in which the reservoir (20) contains two doses of fluid, said spray means (30) and/or said actuator means (40) including dose-fractioning means, so that each time the device is actuated, one dose of fluid is sprayed.

## Patentansprüche

1. Fluidproduktzerstäubungsvorrichtung, aufweisend einen Körper (10), der mit einer Zerstäubungsöffnung (15) versehen ist, einen Vorratsbehälter (20), der zu zerstäubendes Fluidprodukt enthält, Zerstäubungsmittel (30) zum Zerstäuben von in dem Vorratsbehälter (20) enthaltenem Produkt in einer Dosis oder mehreren Dosen, und Betätigungsmittel (40) zum Betätigen der Zerstäubungsmittel (30), wobei der Vorratsbehälter (20) vor der ersten Betätigung der Zerstäubungsvorrichtung dicht verschlossen ist, wobei der Körper (10) Öffnungsmittel (11) für den Vorratsbehälter aufweist, die dazu ausgelegt sind, den Vorratsbehälter (20) bei der Betätigung der Vorrichtung zu öffnen, wobei der Vorratsbehälter (20) eine vom Körper (10) getrennte dichte Einheit bildet, wobei der Vorratsbehälter (20) mit Fluidprodukt befüllt und hermetisch verschlossen ist vor seiner Montage in den Körper (10), wobei der Körper (10) Mittel zur Aufnahme des Vorratsbehälters (13) und Mittel (14) für einen seitlichen Zugang zum seitlichen Montieren und Festlegen des befüllten Vorratsbehälters (20) in dem Körper (10) aufweist, **dadurch gekennzeichnet, dass** die Betätigungsmittel (40) ein seitliches Betätigungselement aufweisen, welches in einer Richtung unterschiedlich von der Verschiebungsrichtung der Zerstäubungsmittel (30) verschiebbar ist.

2. Vorrichtung nach Anspruch 1, wobei die seitlichen Zugangsmittel ein Fenster (14) aufweisen, das in einer Seitenwand des Körpers (10) vorgesehen ist.

3. Vorrichtung nach Anspruch 2, wobei die seitlichen Zugangsmittel zwei diametral gegenüberliegende Fenster (14) aufweisen.

4. Vorrichtung nach einem der vorangehenden Ansprüche, wobei die Aufnahmemittel (13) des Vorratsbehälters Mittel zur Verrastung des Vorratsbehälters (20) in den Körper (10) umfassen.

5. Vorrichtung nach einem der vorangehenden Ansprüche, wobei der Vorratsbehälter (20) durch einen hohlen Schlauch (21) gebildet ist, das durch erste und zweite Stopfen (22, 23) dicht verschlossen ist, die in dem Schlauch (21) angeordnet sind, wobei das Fluidprodukt zwischen den ersten und zweiten Stopfen (22, 23) zu liegen kommt.

6. Vorrichtung nach Anspruch 5, wobei die Zerstäubungsmittel (30) eine Stange (31) aufweisen, die zusammenwirkend mit dem ersten Stopfen (22) des Vorratsbehälters (20) axial verschiebbar ist.

7. Vorrichtung nach Anspruch 5 oder 6, wobei die Öffnungsmittel (11) des Vorratsbehälters Mittel zum Durchbrechen des zweiten Stopfens (23) des Vorratsbehälters (20) aufweisen.

8. Vorrichtung nach einem der vorangehenden Ansprüche, wobei der Körper (10), die Zerstäubungsmittel (30), die Betätigungsmittel (40) und die Öffnungsmittel (11) des Vorratsbehälters zur Bildung einer Einheit zusammengefügt sind, wobei der Vorratsbehälter (20) nach der Befüllung und dem Verschließen mit Stopfen in diese Einheit zusammengefügt werden.

9. Vorrichtung nach einem der vorangehenden Ansprüche, wobei die Mittel (14) für den seitlichen Zugang einen entfernbaren Deckel aufweisen.

10. Vorrichtung nach einem der vorangehenden Ansprüche, wobei der Vorratsbehälter (20) zwei Produktdosen enthält, wobei die Zerstäubungsmittel (30) und/oder die Betätigungsmittel (40) Mittel zum Dosisfraktionieren derart umfassen, dass bei jeder Betätigung der Vorrichtung eine Produktdosis zerstäubt wird.
